Europäisches Patentamt

(19) European Patent Office

Office européen des brevets

(11) Publication number: **0 098 581**
**B1**

(12) **EUROPEAN PATENT SPECIFICATION**

(45) Date of publication of patent specification: **14.05.86**

(51) Int. Cl.⁴: **A 61 K 39/385, A 61 K 39/102**

(21) Application number: **83106552.9**

(22) Date of filing: **05.07.83**

(54) Haemophilus influenzae B polysaccharide exotoxoid conjugate vaccine.

(30) Priority: **06.07.82 US 395743**

(43) Date of publication of application:
**18.01.84 Bulletin 84/03**

(45) Publication of the grant of the patent:
**14.05.86 Bulletin 86/20**

(84) Designated Contracting States:
**AT BE CH DE FR GB IT LI LU NL SE**

(56) References cited:
**US-A-4 356 170**

**INFECTION AND IMMUNITY, vol. 40, no. 1, April 1983, pages 245-256, American Society for Microbiology CHIAYUNG CHU et al.: "Further studies on the immunogenicity of Haemophilus influenzae type b and pneumococcal type 6A polysaccharideprotein conjugates"**

**CHEMICAL ABSTRACTS, vol. 98, no. 15, 11th April 1983, page 469, no. 123940n, Columbus, Ohio, USA R. SCHNEERSON et al.: "Protein-polysaccharide conjugate vaccines"**

**Journal of Experimental Medicine, Vol. 152 (1980) p. 361-376**

(73) Proprietor: **CONNAUGHT LABORATORIES INCORPORATED**
**Swiftwater Pennsylvania 18370 (US)**

(72) Inventor: **Gordon, Lance, Dr.**
**46 Fairview Avenue**
**Mount Pocono Pennsylvania (US)**

(74) Representative: **Werner, Hans-Karsten, Dr. et al**
**Deichmannhaus am Hauptbahnhof**
**D-5000 Köln 1 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The invention relates to a new Haemophilus influenzae b polysaccharide-exotoxoid, exemplified by a polysaccharide-diphtheria toxoid conjugate (PRP-D) vaccine and polysaccharide-tetanus toxoid conjugate (PRP-T) vaccine, the polysaccharide haptens used for making same and the process for producing them.

Schneerson et al., in New Developments with Human and Veterinary Vaccines (1980), pages 77—94, report the induction of increased immunogenicity of the Haemophilus influenzae (Hib) capsular polysaccharide upon subcutaneous injection of a saline solution of Hib polysaccharide covalently bonded to protein conjugates. Adipic hydrazide derivatives of albumin, hemocyanin, and diphtheria toxin were prepared. Schneerson et al. report in their article Protein-Polysaccharide Conjugate Vaccines (1982), pages 265—272 the use of several proteins, including diphtheria toxin, to prepare conjugates with various bacterial capsular polysaccharides for use as "T-cell dependent" antigens for subcutaneous saline injections in laboratory animals. About 85% of the protein was converted to the conjugate and about 30% of the polysaccharide was bound by the void volume after passage of the conjugate through a 4B Sepharose® column. Cyanogen bromide activation of the Hib polysaccharide and adipic acid dihydrazide derivative of the protein was used.

Schneerson et al. report in Bacterial Capsular Polysaccharide Conjugates in L. Weinstein and B. N. Fields (ed.) Seminars in infections disease vol. 4, Bacterial vaccines, New York (1982), pages 311—321 a method for covalently bonding Hib capsular polysaccharide to proteins in order to increase the polysaccharide's immunogenicity. AH derivatives of BSA, HSA, diphtheria toxin, and hemocyanin were used as carrier proteins.

The "Journal of Experimental Medicine" Vol. 152 (1980), pages 361—376 discloses a method for covalently bonding Haemophilus influenzae type b capsular polysaccharide to several proteins. This method relies upon the use of adipic dihydrazide as a spacer between the capsular polysaccharide and the carrier protein.

None of the above-discussed articles used toxoids, and the authors of these articles do not mention a step to adjust the molecular weight of the hapten, avoidance of the use of calcium, removal of excess cyanogen halide to avoid excessive cross-linking, and recovery of 70 to 100% on starting PRP.

Schneerson et al. in Prog Allergy, volume 33, pages 144—178 (1982) studied the preparation and effect of vaccine composed of polysaccharide covalently bound to a "T-dependent" carrier protein. The adipic hydrazide derivatives of diphtheria toxins or tetanus toxoid were covalently reacted with cyanogen bromide-treated Hib polysaccharide and the product was dialyzed, passed over a 4B Sepharose® column, sterile filtered, and stored as a sterile saline solution.

Chu et al. in Infection and Immunity (1983) pages 245—256 teach the preparation of conjugates by coupling AH derivatives of Hib polysaccharides with tetanus toxoid or hemocyanin. The polysaccharide was activated with CNBr, reacted with ADH, and dialyzed, followed by gel filtration and lyophilization. The AH polysaccharide derivative and the protein were then coupled. The yield of Hib-tetanus toxoid conjugates was 62% when the reactants were 30 mg/ml. The authors of the two above-discussed articles do not mention a step to adjust the molecular weight of the hapten, avoidance of the use of calcium, or removal of excess cyanogen halide to avoid excessive cross-linking.

The present invention provides a water-soluble, heat resistant, Haemophilus influenzae b polysaccharide-exotoxoid conjugate characterized in that said conjugate is capable of producing T-cell dependent antibody response to polysaccharide from Haemophilus influenzae b,

the range of molecular size is above 140,000 and below 4,500,000 dalton as referenced to dextran standard by gel permeation chromatography, and

the ratio of a ribose/protein is between 0.25 and 1.00, and said conjugate is prepared by mixing a 4—8 carbon dicarboxylic acid hydrazide derivatized toxoid in a

cyanogen halide-free solution with a cyanogen halide activated capsular Haemophilus influenzae b polysaccharide consisting of equimolar amounts of ribose, ribitol and phosphate, which had previously been sized by heating until more than 60% of the polysaccharide was adjusted to a molecular size between 200,000 and 2,000,000 dalton as referenced to dextran standard by gel permeation chromatography.

Such a conjugate is especially valuable for use in prevention of Haemophilus infections in infants and young children.

The invention also makes available a conjugate vaccine useful for immunization against diseases such as diphtheria and tetanus. The potency in humans of such conjugates is greater than that of the conventional diphtheria and tetanus toxoid preparations. The method, by careful control and removal of excess activator, provides a polysaccharide conjugate constructed of multiple molecules of exotoxoid attached to single molecules of polysaccharide, linked through a spacer, and substantially free from cross-linked or polymeric derivatives. The formula may be generally represented as PRP-$X_n$ wherein X is the toxoid moiety and n is an integer smaller than 20, a typical average being 7—8. (The diphtheria toxoid conjugate is referred to herein as PRP-D, the tetanus conjugate as PRP-T without use of the integer n).

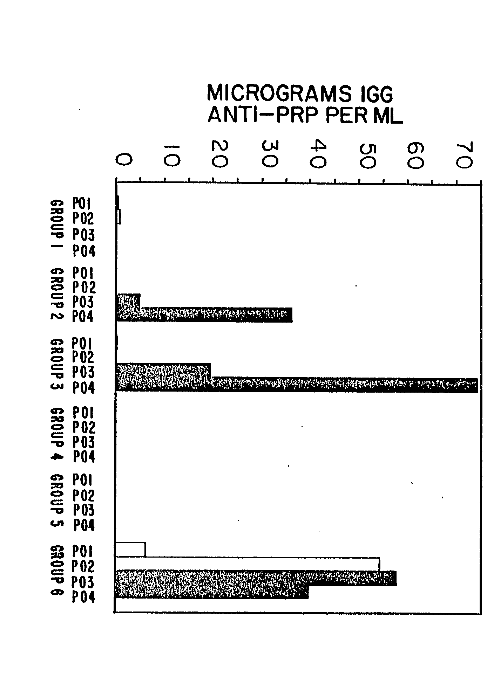

Figure 1 demonstrates the immunogenicity of Haemophilus influenzae b capsular polysaccharide, i.e., the anti-PRP response. This bar graph presents the data according to the protocol of Example V.

The development of stable humoral immunity requires the recognition of foreign material by at least two separate sets of lymphocytes. These sets are the B-lymphocytes, which are the precursors of antibody forming cells, and the T-lymphocytes which modulate the function of B-cells. While some antigens, including several polysaccharides, are capable of directly stimulating B-cells to produce antibodies (T-independent antigens), most antigens (T-dependent) must be presented to the B-cell by a T-lymphocyte. In the case of the vaccines of this invention, the exotoxoid portion of the vaccine is recognized by the T-cell system. Since the protein carries both its own antigenic determinants and the covalently bound PRP hapten, both sets of determinants should be presented by T-cells to B-cells. The result of administration of this carrier-hapten conjugate preparation is that PRP is presented as a T-dependent immunogen. A T-dependent presentation of PRP induces protective immunity in infants, the target population at greatest risk. The conjugate vaccines of this invention are therefore of special value in small infants. Administration of such conjugate to women induces a high proportion of IgG antibody to the vaccine antigens, which penetrates the placental barrier during pregnancy, and thus affords protection to the infant from birth.

T-independent antigens induce B-cells to terminally differentiate into antibody secreting cells (plasma cells), while T-dependent responses are considerably more complex. After receiving a T-dependent stimulus, the B-cell population enters not only antibody production, but also proliferation and maturation. There results an increase in the number of B-cells making antibodies to PRP and an increase in the number of B-cells capable of responding to a second exposure to PRP. Repeated immunization results in further increases in the number of PRP specific B-cells and, consequently, higher antibody titers, a booster response. In summary, while T-independent responses are limited by the number of responsive B-cells, T-dependent responses result in an increase in the total number of antigen responsive cells.

The PRP-exotoxoid vaccine of this invention has been shown to function as a T-dependent immunogen in laboratory animals. All animals in a group of rabbits serially immunized with a standard dose of PRP-D showed booster responses. In addition, primary immunizations with the carrier proteins used in the conjugate, e.g. diphtheria toxoid for PRP-D, were shown to augment the initial response to the PRP component of the PRP-exotoxoid conjugate. A similar augmentation of the PRP response was not seen in animals primed with an exotoxoid other than that used in the conjugate.

The vaccine of this invention is a PRP-exotoxoid hapten-carrier conjugate. In such vaccines, the antigenic but weakly immunogenic hapten molecule (PRP) contributes a new antigenic specificity to highly immunogenic carrier molecules such as diphtheria (D) or tetanus toxoid (T).

The purified diphtheria toxoid (D) used as carrier in the preparation is a commercial exotoxoid modified (derivatized) by the attachment of a 4—8 carbon spacer molecule, such as adipic acid dihydrazide (ADH), using the water-soluble carbodiimide condensation method. The modified exotoxoid, typically the adipic hydrazide derivative X-AH, is then free from unreacted ADH. This is a soluble product, substantially free of cross-linking which is verified by lack of a substantial increase in molecular size as determined by gel chromatography and polyacrylamide gel electrophoresis.

The capsular polysaccharide of Haemophilus influenzae b is prepared from commercial sources such as used for licensed polysaccharide vaccines and is obtainable from Connaught Laboratories. However, while the polysaccharide is conventionally purified as a calcium salt, the use of calcium ions is avoided because they interfere with the use of carbonate buffer in the conjugation procedure. The molecular size of the polysaccharide is then adjusted by heating until the desired dimension for the hapten is obtained. Typically, heating of the liquid polysaccharide for 15 minutes at 100°C suffices to assure that less than 20% of the molecules are of a molecular size smaller than 200,000 dalton and less than 20% of a molecular size greater than 2,000,000 dalton. This sizing operation is important to obtain a proper PRP-D or PRP-T conjugate.

The sized polysaccharide thus obtained is activated with an activator to create an electrophilic group on the polysaccharide such as a cyanogen halide or sodium borohydride. A typical activator used is cyanogen bromide. Unreacted activator is exhaustively removed because, if there is a substantial residue, it causes cross-linking of the protein in the following reaction mixture. The resulting cross-linked product traps polysaccharide, producing a lower yield of vaccine and a higher molecular size conjugate differing in chemical properties and solubility from the conjugate produced herein and a vaccine which lacks the desirable proportion of the vaccine of this invention.

The activated PRP and X-AH are then combined and allowed to react in the cold. Some of the hydrazide groups on the derivatized exotoxoid react with the activated sites on PRP to form covalent bonds. The product is PRP covalently bound to derivatized exotoxoid through a 4—8 carbon bridge. This reaction product is purified by gel permeation chromatography to remove any unreacted protein and low molecular size contaminants. The typical molecular size of the principal fraction is about 675,000 dalton relative to dextran standard. A typical range for relative molecular size is 140,000 dalton to 4,500,000 dalton.

A preservative such as thimerosal is added, in the case of thimerosal to a final concentration of 1:10,000. The bulk concentrate is filtered through a 0.2 μm membrane filter and stored in the cold.

The PRP-exotoxoid conjugation product is heat resistant and water soluble. The lack of cross-linking is verified by lack of a substantial increase in molecular size from that of the starting polysaccharide as determined by gel permeation chromatography and by polyacrylamide gel electrophoresis. Heat stability assures a long shelf-life and a stable product even in unfavorable climates.

The reaction can be represented schematically as two steps;

3

$$(1)\ PRP+CNBr \rightarrow PRP*$$

$$(2)\ PRP*+nX \rightarrow PRP(-X)n$$

If the cyanogen halide removal is not efficiently carried out, there occur besides conjugation of the activated PRP and exotoxoid to form PRP(−X)n, such undesirable reactions as formation of X−X of multiply linked derivatives, e.g.,

$$PRP*+CNBr \rightarrow nX+XX\text{——}PRP\text{—}X$$
$$X\text{-}X\text{-}PRP$$

A typical human dose of PRP-exotoxoid conjugate vaccine for subcutaneous or intradermal injection consists of 0.5 ml containing 10 micrograms of ribose or the approximate equivalent of 40 micrograms of the conjugate product. Ampoule solutions are prepared by dilution of the bulk concentrate with phosphate buffered saline solution using thimerosal as a preservative.

The product induces antibody formation in humans to both the PRP and exotoxoid component.

The following examples are provided for purposes of illustrating the invention in further detail. They are not to be construed as limiting the invention in spirit or in scope.

Example I: Preparation of PRP

A. Organism

Capsular polyribosyl-ribitol-phosphate (PRP) of *Haemophilus influenzae* b was prepared from the Eagan strain. The culture was repeatedly transferred and a lyophilized seed prepared. From this lyophilized seed one additional transfer was made to prepare wet working seeds (stored at −60°C). Fermenter lots of bacterial cells were prepared from the wet working seed.

Culture purity is determined by the following criteria:

1. negative Gram stain characteristics;
2. growth on agar containing NAD (diphosphopyridine nucleotide) and Hemin (Bovine Type I crystalline salt of ferriheme);
3. failure to grow on agar without NAD or Hemin; and
4. agglutination by specific antisera (type b *Haemophilus influenzae* b, Hyland Laboratories).

B. Cultivation and media

For subculturing the bacterium, i.e., preceding inoculation of a fermenter, BHI Agar (per liter: 37 g BHI Difco, 15 g Bacto Agar Difco, 0.6 ml 1% NAD Sigma-Grade III and 6 ml of 0.2% Hemin (Sigma-Bovine Type 1) was employed. Cells (20 hours) washed from an agar surface are used to inoculate *Haemophilus influenzae* b (Hib) liquid media (1 liter aliquots); these cultures are incubated with shaking until the bacterium reaches the log phase of its growth cycle. At this time, 2 liters of culture are used to inoculate each 40 liters of liquid Hib media in a fermenter and 300 ml 8% UCON (Union Carbide lubricant) is added. After 16 to 18 hours, the fermenter culture is ready for harvest.

The composition of the Hib liquid media per 1000 ml is:

| | |
|---|---|
| Yeast extract dialysate, Difco | 5.0 g |
| Casamino acids, Difco | 22.5 g |
| Sodium phosphate, dibasic | 14.4 g |
| Dextrose | 5.59 g |
| Hemin | 20 g |
| Ammonium hydroxide (30%) | 0.1534 ml |
| NAD 1% | 0.6 ml |

When harvesting a fermenter, culture purity is determined by appropriate Gram staining and culturing techniques (see A 1—4 above). Cetavlon® (hexadecyltrimethylammonium bromide) is added to the culture to a final concentration of 0.1%. After 30 minutes, at which time the bacteria have been inactivated, the solid paste is collected by centrifugation. The wet paste is stored at −70°C until further processing.

C. Purified polysaccharide

The extraction and subsequent purification of the PRP is carried out using the following procedure:

1. Dissociation from detergent

For each gram wet weight of paste, 10 ml of 0.4 M NaCl is added. The suspension is mixed in a commercial blender for 30 seconds. The mixture is centrifuged for 15 minutes at 17,000 Xg in the cold (4°C). The supernatant is collected and ethanol is added to a concentration of 25%. This material is then centrifuged for 2 hours at 17,000 Xg (4°C) and the supernatant saved. Ethanol, at four times the volume of the supernatant, is added and the material held overnight at 4°C.

2. Removal of nucleic acids

The material is centrifuged for 5 minutes at 2800 Xg (4°C). The sediment is collected and resuspended in Tris-$MgSO_4$ buffer at one-fourth the volume originally used to extract the paste. The composition of the Tris buffer is as follows per liter of distilled water:

| | |
|---|---|
| tris-hydroxymethylamino-methane (Sigma) | 6 g |
| $MgSO_4 \cdot 7\ H_2O$ | 246 mg |
| thimerosal (Elanco) | 50 mg |

The pH is adjusted to 7.0±0.2 with concentrated hydrochloric acid.

Deoxyribonuclease I 1.5 mg (Sigma D-0876) and ribonuclease-A 0.75 mg (Sigma-Type 1-AS, R-5503) per 100 g of original wet paste are added. The material is placed in a dialysis bag and incubated for 18 hours at 37°C versus 18 liters of Tris-$MgSO_4$ buffer.

3. Removal of proteins

The material is further processed to remove protein components by adding an equal volume of phenol-acetate solution (135 ml of 10 percent (w/v) sodium acetate combined with 454 g of phenol). The material is then shaken for 30 minutes (4°C), centrifuged for 15 minutes at 17,000 Xg and the aqueous phase collected. Two additional phenol extractions are conducted followed by dialysis of the last aqueous phase versus distilled water.

The material at this stage constitutes the bulk liquid capsular polysaccharide (PRP) and is stored at −20°C until further processing (Section D below).

4. Assessment of quality of polysaccharide

The quality of the bulk PRP is judged based on the analysis of a liquid sample that is removed. Ethanol is added (4 volumes), then $CaCl_2$ (final concentration of 0.02M) and the PRP is precipitated. The PRP is sedimented by centrifugation and dried under vacuum over a desiccant. A thermogravimetric analysis (TGA) is used to determine the moisture content. Further analyses are calculated on a dry weight basis.

Criteria for acceptance include:

(a) analysis of ribose (Orcinol method): greater than 30 percent,

(b) analysis of protein (Lowry method): less than 1 percent,

(c) analysis of nucleic acids (U.V. adsorbance): less than 1 percent, and

(d) precipitation with specific immune sera (counterimmunoelectrophoresis method).

In addition, the molecular size is determined by suitable gel permeation chromatography. The polysaccharide is monitored for endotoxin by Limulus Lysate testing and by rabbit pyrogenicity testing.

A typical lot has the following characteristics:

(a) Protein content, 0.5%

(b) Nucleic acid content, 0.35%

(c) Residual bovine antigens: No contamination of the purified PRP by bovine RNA-ase and DNA-ase used in preparation of the polysaccharide, as measured by radioimmunoassay.

(d) Endotoxin content was measured by the Limulus Amoebocyte Lysate Assay (LAL): 200 ng/mg PRP.

(e) Kd on CL-4B Sepharose®: 0.30. A value of 0.30 corresponds to an approximate molecular weight of 1,125,000 relative to dextran standards.

D. Preparation of polysaccharide (PRP) reagent

The polysaccharide is thus purified as a liquid, but calcium is not used in the purification procedure. (Conventional polysaccharide purification yields a calcium salt, but calcium can combine with the carbonate buffer used hereinbelow during conjugation to form a precipitate.)

The size of the polysaccharide is adjusted by heating at 100°C for a time proportional to the degree of size change needed. Size of the polysaccharide is adjusted so that less than 20% elutes from a CL-4B Sepharose® column in the void volume, and less than 20% elutes with a Kd greater than 0.5. The principal fraction has a molecular size of 200,000 to 2,000,000 dalton.

Example II. Activation of PRP

A. This polysaccharide is cooled on an ice bath to 4°C in a reaction vessel equipped with a magnetic stirrer. The initial volume of PRP at the concentration of 25 mg/ml (20—30 mg/ml range) in distilled water is recorded. Then sodium chloride is added to a concentration of 0.85%.

B. The pH of the resulting solution of the sodium salt of the polysaccharide is raised to 10.5—11.0 by addition of 1N sodium hydroxide. (This range is chosen because at a lower pH there is less reaction with cyanogen bromide and at higher pH the polysaccharide breaks down.)

C. Dry cyanogen bromide is dissolved in 0.005N sodium bicarbonate buffer of pH 10.5—11.0 and immediately (within 10 minutes of preparation) is added to the reaction vessel in a proportion of 0.4 mg/mg PRP.

D. The pH of the mixture is adjusted to and maintained at 10.5—11.0 for 6 minutes by addition of sodium hydroxide.

E. The pH is then dropped to 6.0 with 1N HCl. (Acid pH stabilizes the activated sites created on the polysaccharide by cyanogen bromide. Lowering the pH further results in hydrolysis of the PRP.)

G. There is added an equal volume of saline, pH 6.0, pre-chilled to 4°C.

H. The cyanogen bromide-polysaccharide mixture is transferred to a concentrator apparatus and concentrated to the initial volume recorded at step A.

I. Steps G and H are repeated at total of 10 times. Thus about 99.9% of the unconsumed cyanogen bromide is removed while the polysaccharide concentration is maintained at 25 mg/ml. If the cyanogen bromide is not removed, it reacts with the diphtheria exotoxoid used below.

Example III. Preparation of D-AH carrier

A. Commercial diphtheria exotoxoid (D) in distilled water is concentrated to 20—40 mg/ml, typically 35 mg/ml over a membrane that retains molecules over 10,000 dalton in a positive pressure stirred concentrator.

B. A dry mixture of 8 mg adipic acid dihydrazide (ADH) per mg protein and 0.75 mg 1-ethyl-3-(3-dimethylaminopropyl)carbodiimide (EDAC) per mg protein are placed in a reaction vessel equipped with an efficient stirrer and a pH probe to permit monitoring and control of pH.

C. The diphtheria exotoxoid is then added to the reaction vessel making the proportion of the reactants:

| diphtheria exotoxoid | =35 mg protein/ml |
|---|---|
| ADH | =8.0 mg/mg protein |
| EDAC | =0.75 mg/mg protein |

(The use of acetate buffer on sequential addition of ADH and EDAC results in a persistent flocculent precipitate. The reactants have a sufficient buffering capacity of their own.)

D. The pH is immediately adjusted to 4.7 and maintained at $4.7 \pm 0.2$ for a minimum of 2 hours.

1. The course of the chemical reaction can be followed on the recorder of the pH controller. If necessary, the reaction is allowed to proceed past 2 hours until the pH is stable for at least 15 minutes, (i.e., 15 minutes without needing HCl addition to control pH).
2. The amount of HCl consumed is recorded as a process check.
3. Temperature change in the reaction vessel is also monitored.

E. The reaction product is dialyzed at 4°C against two changes of saline, a minimum of 100 volumes and 8 hours per change.

F. It is then dialyzed against two changes of phosphate buffered saline, minimum volume, time and temperature as for step E.

G. The product (D-AH) is concentrated back to 25 mg/ml protein with a positive pressure apparatus and 10,000 MW membrane.

H. The concentrate is sterile filtered (0.2 μm) and stored at 4°C.

Assay of a sample of a typical lot of such D-AH carrier showed a ratio of 38.3 microgram ADH/mg DT. Chromatography of that sample showed a Kd value on Cl-4B Sepharose® of 0.75 which corresponds to an approximate molecular weight of 139,000 relative to protein standards.

Example IV. Formation of the covalent Polysaccharide-diphtheria toxoid conjugate (PRP-D)

A. The diphtheria toxoid adipic acid hydrazide carrier (D-AH) of Example III, at a concentration of 25 mg/ml, is treated with sodium bicarbonate to a concentration of 0.5M and the pH is adjusted to 8.5. (Significantly lower salt concentrations result in the formation of a gel in the final reaction mixture with activated polysaccharide.)

B. In a reaction vessel which can be sealed, an equal amount of the washed PRP solution produced in

6

Example II is added and the pH should be stable at 8.4—8.6. (If CNBr has not been removed, the pH will drift rapidly downward and a copious precipitate will form. This reaction occurs even in the absence of polysaccharide. There should not be a significant change of the physical appearance of the reactants on combination.)

C. The reaction mixture is tumbled for 15—18 hours at 4°C.

D. The conjugate is purified by gel permeation chromatography on Sephacryl®-300 equilibrated in phosphate buffered saline to remove unreacted protein and material of molecular size less than 140,000 dalton in size. (If the starting polysaccharide is too small it will not be possible to separate the conjugate from free protein in this manner.)

E. Samples of this purified conjugate are removed for chemical analysis, described in the following section of this example.

F. Thimerosal is added to the purified conjugate to a concentration of 1:10,000 and the product is stored at 4°C until analysis.

G. The product is 0.2 µm sterile filtered. (If the polysaccharide is not sized as described in Example I(D), i.e., if it is oversize, the resulting conjugate will not be filterable.)

Analysis Tests performed on the bulk concentrate of Example IV gave the following results:

a) Ribose content: 156.5 microgram/ml.

(For the calculation of PRP values in Example V, a conversion factor of 2 is used to calculate a nominal polysaccharide concentration based on the empirically determined ribose concentration.)

b) Protein content: 330 microgram/ml.

c) Ribose/protein ratio: 0.47 (limits 0.25—1.0)

d) Chromatographic analysis on Sepharose® CL-2B gave the chromatographic profile which shows a homogeneous distribution of conjugate molecules as a single peak.

e) Kd (polysaccharide): 0.36 using Sepharose® Cl-4B, 0.77 using Cl-2B.

Determined by individual fraction ribose assay for PRP. A value of 0.36 on Cl-4B corresponds to an approximate molecular size of 674,000 relative to dextran standards.

f) Kd (protein): 0.34 using Cl-4B; 0.71 using Cl-2B.

Determined by individual fraction Lowry assay for protein. The change in the Kd value of the diphtheria exotoxoid from 0.75 to 0.34 on Cl-4B shows that the conjugation of protein with polysaccharide forms a molecule which is chromatographically different from either raw material.

g) Free Protein: Less than 5%.

Free protein represents derivatized diphtheria carrier protein which has not been bound to PRP. It is determined by comparing the amount of protein that elutes in the position of a diphtheria exotoxoid sample relative to the total eluted protein.

h) Endotoxin Content: 1 µg/ml

Endotoxin was quantitated by the Limulus Amoebocyte Lysate Assay (LAL). The endotoxin content amounts to 64 ng per 10 µg ribose human dose of PRP-D.

i) Pyrogenicity:

The bulk concentrate meets the United States standards for non-pyrogenicity using a weight equivalent (human) dose of 0.15 µg ribose per milliliter per kilogram body weight of rabbit.

j) Polyacrylamide Gel Electrophoresis (PAGE):

PAGE analysis was performed to obtain supporting evidence of purity and covalent bonding between polysaccharide and protein. While free carrier bands just over halfway into the rod gel, at approximately the position of catalase (60,000 MW), the PRP-D conjugate was not able to enter the gel (prior to the position of thyroglobulin, 330,000 MW). PRP-D showed a single band at the origin.

k) Cyanogen Bromide:

While cyanogen bromide (CNBr) is used in the first steps before preparing a PRP-D conjugate, it is subsequently excluded from the product. Several steps of the process contribute to the reduction of CNBr content. However, final purification of the vaccine by gel permeation chromatography removes any contaminants below 100,000 MW. This purification precludes contamination with residual traces of free CNBr or its degradation products.

l) Heat Stability: A study for heat resistance of PRP-D conjugate vaccine was performed. The bulk material was concentrated 40 fold and three 3 ml samples were taken. The first was kept at 4°C water bath for 16 hours, and the third was heated in a 100°C water bath for 30 minutes. The tests performed were for protein and ribose content, gel permeation chromatography with Sepharose® Cl-4B and SDS-polyacrylamide gel electrophoresis (PAGE).

The results showed no significant change in ribose or protein content between the samples compared to the results in tests (a) and (b) above. The chromatographic analyses showed a minor decrease in the molecular size of the conjugate material as conditions were made increasingly harsh. However, upon fractional analysis of these samples by measuring absorbance at 254 nm, the material maintained only one peak coinciding with the polysaccharide peak and no free protein peak appeared; the results are thus comparable to those in (d) and (g) above. This demonstrates that the linking between the protein and polysaccharide did not break but the decrease in molecular size was due to the breaking of bonds within the linear polysaccharide. This was further demonstrated by comparison of PAGE analysis of these samples to the results described under (j) above; free protein was not detectable in any of the samples even in the presence of a detergent (sodium dodecyl sulfate). This strongly affirms the covalency of the derivatized protein-polysaccharide linkage and its stability through high temperature treatment.

Example V. PRP-D Immunogenicity testing

This experiment was designed to show T-cell dependency as evidenced by: carrier dependency, carrier specificity, booster effect, and change in Ig class. This experiment was carried out in two parts: 1) an initial priming sequence of two injections; 2) a challenge sequence of two injections.

Materials:
   1. PRP-D Bulk concentrate, Example 4.
   2. Tetanus Toxoid (T).
   3. Diphtheria Toxoid (D).
   4. *H. influenzae* b capsular polysaccular (PRP).
   5. PRP/D, a mixture of 20 µg PRP (10 µg ribose) and 20 µg D.
   6. PRP/D-AH, a mixture of 20 µg PRP (10 µg ribose) and 20 µg D-AH.

Method:
   All immunizations were administered subcutaneously without adjuvant in 1 ml volumes. All doses containing PRP were adjusted to 10 µg ribose per 1 ml dose. Unconjugated exotoxoid doses were adjusted to 20 µg protein per 1 ml dose. A rotating schedule was used with immunizations spaced 14 days apart. Each immunization was followed by a bleed 10 days later. All preparations were diluted in phosphate buffered saline containing 0.01% thimerosal. Aliquots were prepared as four dose vials, and stored at −20°C. Three rabbits were immunized in each group.

Serology:
   Sera were assayed by the solid phase radioimmunoassay (SPRIA) for anti-PRP, anti-D-AH, anti-D, and anti-T as indicated. Antibodies levels were quantitated as microgram IgG and IgM per ml.

Protocol:

| Group | Primary | Secondary | PRP | SPRIA<br>DT-AH | DT | TT |
|---|---|---|---|---|---|---|
| 1 | PRP | PRP | + | | | |
| 2 | T | PRP-D | + | + | + | + |
| 3 | D | PRP-D | + | + | + | + |
| 4 | PRP/D | PRP/D | + | | + | |
| 5 | PRP/D-AH | PRP/D-AH | + | + | | |
| 6 | PRP-D | PRP-D | + | + | + | |

Schedule:
   Rabbits were pre-bled and immunized according to group every 14 days. Each immunization was followed by a post-bleed 10 days later. The first two injections were made with primary immunogen, while the third and fourth injections were made with the secondary immunogen.

   Anti-PRP response is demonstrated in Figure 1. The mean level of IgG antibody to *Haemophilus influenzae* b capsular polysaccharide in the six experimental groups of rabbits (three animals per group) are graphically illustrated. Groups are labelled on this figure in accordance with the above protocol. Prebleed levels were less than 1 µg/ml for all rabbits and are not shown in this figure for clarity.

   Open bars represent the level of IgG following two successive injections with the primary immunogen. Solid bars show the IgG levels following the third and fourth injections which were with the secondary or challenge immunogens.

   IgG responses were seen only following immunization with the PRP-D conjugate vaccine. Priming the rabbits in group 3 with diphtheria toxoid accelerated the response to PRP-D, while priming the tetanus toxoid (group 2) had no effect.

TETANUS TOXOID
Example VI. Preparation of T-AH carrier and formation of covalent
Polysaccharide-tetanus toxoid conjugate (PRP-T)
   In the procedure of Example III, commercial tetanus toxoid is used in the place of diphtheria toxoid to produce the adipic acid hydrazide derivative.

Assay of a sample of a typical lot of T-AH carrier showed a ratio of 53.9 microgram ADH/mgT. Chromatography of that sample showed a Kd value of Cl-4B Sepharose® of 0.66 which corresponds to an approximate molecular weight of 227,000 relative to protein standards.

Polysaccharide conjugates with the adipic hydrazide derivate of tetanus toxoid are formed using the same procedures as for D-AH, as described in Example IV.

Analysis of a typical lot of PRP-T showed 142.6 micrograms of polysaccharide and 260 micrograms of protein per ml. The conjugate was soluble and 0.2 μm filterable. When chromatographed on CL-4B the protein component demonstrated a Kd of 0.30 and the polysaccharide component a Kd of 0.37.

TABLE 1
Anti-PRP response
Solid phase radioimmunoassay for IgM and IgH

| : | : | PRP | | Diphtheria toxoid | | Tetanus toxoid | |
|---|---|---|---|---|---|---|---|
| : | : | IgG* | IgM | IgG | IgM | IgG | IgM |
| Group 1 Pre | | 0.0 | 0 | ND% | | ND | |
| | Post 1 | 0.33 "0.58 | 0 | | | | |
| | Post 2 | 0.8 "1.39 | 0 | | | | |
| | Post 3 | 0 | 0 | | | | |
| | Post 4 | 0 | 0 | | | | |
| Group 2 Pre | | 0 | 0 | 0 | 0 | 0.5 "0.87 | 0 |
| | Post 1 | 0 | 0 | 0 | 0 | 25.1 "15.10 | 113.1 "129.54 |
| | Post 2 | 0 | 0 | 0 | 0 | 112.00 "0 | 44.60 "36.90 |
| | Post 3 | 4.82 "2.59 | 26.3 "45.55 | 0 | 0 | 112.00 "0 | 8.50 "14.72 |
| | Post 4 | 36.1 "17.43 | 74.57 "28.81 | 0 | 0 | 151.60 "52.64 | 2.00 "3.46 |
| Group 3 Pre | | 0.2 "0.35 | 0 | 0 | 27.07 "46.88 | 0.8 "1.39 | 65.00 "112.58 |
| | Post 1 | 0.2 "0.35 | 0 | 0 | 0 | 0.8 "1.39 | 0 |
| | Post 2 | 0 | 14.6 "25.29 | 6.47 "5.98 | 0 | 0.67 "0.59 | 0 |
| | Post 3 | 19.4 "13.4 | 130.33 "89.49 | 10.63 "8.08 | 0 | 0.37 "0.65 | 0 |
| | Post 4 | 74.2 "76.73 | 82.63 "5.16 | 29.03 "26.59 | 0 | 1.30 "2.25 | 0 |

# 0 098 581

### TABLE 1 Cont'd

| | | PRP | | Diphtheria toxoid | | Tetanus toxoid | |
|---|---|---|---|---|---|---|---|
| : | : | IgG* : | IgM | : IgG : | IgM | : IgG : | IgM |
| Group 4 | Pre | 0 | 0 | 0 | 21.77 ¨18.85 | ND | |
| | Post 1 | 0 | 15.6 ¨27.02 | 2.67 ¨0.85 | 30.87 ¨27.14 | | |
| | Post 2 | 0 | 0 | 37.2 ¨10.94 | 26.2 ¨24.30 | | |
| | Post 3 | 0 | 0 | 210.00 ¨0 | 19.63 ¨17.06 | | |
| | Post 4 | 0 | 0 | 298.27 ¨207.70 | 0 | | |
| Group 5 | Pre | 0 | 0 | ND | | | |
| | Post 1 | 0 | 0 | | | | |
| | Post 2 | 0 | 0 | | | | |
| | Post 3 | 0 | 0 | | | | |
| | Post 4 | 0 | 0 | | | | |
| Group 6 | Pre | 0 | 0 | 0 | 0 | | |
| | Post 1 | 6.23 ¨2.56 | 135.00 ¨81.41 | 0 | 0 | | |
| | Post 2 | 54.2 ¨14.73 | 182.00 ¨0 | 0.93 ¨1.62 | 0 | | |
| | Post 3 | 57.6 ¨22.91 | 182.00 ¨0 | 3.05 ¨4.31 | 0 | | |
| | Post 4 | 39.6 ¨25.17 | 91.20 ¨0 | 5.95 ¨8.41 | 0 | | |

\* IgG and IgM are expressed as µg antibody per ml of serum. All value are mean and standard error.

\# Ig levels below the sensitivity of the assay were assigned a value of zero (0). If levels higher than the range of the assay *this* were assigned a value equal to the upper limit of the assay.

% ND=Not Done.

**Claims**

1. A water-soluble, heat resistant, Haemophilus influenzae b polysaccharide-exotoxoid conjugate characterized in that said conjugate is capable of producing T-cell dependent antibody response to polysaccharide from Haemophilus influenzae b,

the range of molecular size is above 140,000 and below 4,500,000 dalton as referenced to dextran standard by gel permeation chromatography, and

the ratio of a ribose/protein is between 0.25 and 1.00, and said conjugate is prepared by mixing a 4—8 carbon dicarboxylic acid hydrazide derivatized toxoid in a

cyanogen halide-free solution with a cyanogen halide activated capsular Haemophilus influenzae b polysaccharide consisting of equimolar amounts of ribose, ribitol and phosphate, which had previously been sized by heating until more than 60% of the polysaccharide was adjusted to a molecular size between 200,000 and 2,000,000 dalton as referenced to dextran standard by gel permeation chromatography.

2. A conjugate of claim 1, wherein said dicarboxylic acid is adipic acid.

3. A conjugate of claim 1, wherein said toxoid is diphtheria toxoid.
4. A conjugate of claim 1, wherein said toxoid is tetanus toxoid.
5. A conjugate of claim 1, wherein said halide is the bromide.
6. A process for producing a toxoid conjugate according to claims 1 to 5, characterized in that said process comprises:

(a) heating a capsular polysaccharide of Haemophilus influenzae b consisting of equimolar amounts parts of ribose, ribitol and phosphate until less than 20% is of a molecular size below 200,000 dalton and less than 20% is of a size above 2,000,000 dalton as referenced to dextran standard by gel permeation chromatography;

(b) activation of the resulting sized polysaccharide with cyanogen bromide;

(c) removal of substantially all the unreacted cyanogen bromide; and

(d) reaction of the activated product with a 4—8 carbon dicarboxylic acid hydrazide derivatized exotoxoid.

7. A hapten free from protein and nucleic acid prepared from capsular Haemophilus influenzae b polysaccharide, consisting of equimolar amounts of ribose, ribitol and phosphate, by heating until less than 20% is of a molecular size is below 200,000 dalton and less than 20% is of molecular size greater than 2,000,000 dalton as referenced to dextran standard by gel permeation chromatography.

8. A hapten of claim 7 wherein the polysaccharide is present as the sodium salt.

**Patentansprüche**

1. Wasserlösliches, hitzebeständiges Haemophilus Influenzae B Polysaccharid-Exotoxoid-Konjugat, dadurch gekennzeichnet, daß

das Konjugat befähigt ist, eine T-zellenabhängige Antikörper-Antwort auf Polysaccharid aus Haemophilus Influenzae B zu erzeugen,

der Bereich der Molekül-Größe oberhalb von 140 000 und unterhalb von 4 500 000 Dalton, bestimmt durch Gelpermeationschromatographie und bezogen auf den Dextran-Standard, liegt und das Verhältnis Ribose/Protein zwischen 0,25 und 1,00 liegt und

das Konjugat hergestellt wird durch Vermischen eines mittels eines Hydrazids einer Dicarbonsäure mit 4 bis 8 Kohlenstoff-Atomen derivatisierten Toxoids in einer Lösung, die frei von Cyanhalogeniden ist, mit einem durch Cyanhalogenid aktivierten kapsulären Haemophilus Influenzae B Polysaccharid, bestehend aus äquimolaren Mengen Ribose, Ribit und Phosphat, das vorher durch Erhitzen kalibriert worden war, bis mehr als 60% des Polysaccharids auf eine Molekülgröße zwischen 200 000 und 2 000 000 Dalton, bestimmt durch Gelpermeationschromatographie und bezogen auf den Dextran-Standard, eingestellt worden waren.

2. Konjugat nach Anspruch 1, dadurch gekennzeichnet, daß die Dicarbonsäure Adipinsäure ist.

3. Konjugat nach Anspruch 1, dadurch gekennzeichnet, daß das Toxoid Diphtherie-Toxoid ist.

4. Konjugat nach Anspruch 1, dadurch gekennzeichnet, daß das Toxoid Tetanus-Toxoid ist.

5. Konjugat nach Anspruch 1, dadurch gekennzeichnet, daß das Halogenid das Bromid ist.

6. Verfahren zur Herstellung eines Toxoid-Konjugats nach Ansprüchen 1 bis 5, dadurch gekennzeichnet, daß das Verfahren umfaßt:

(a) das Erhitzen eines kapsulären Polysaccharids von Haemophilus Influenzae B, bestehend aus äquimolaren Mengen Ribose, Ribit und Phosphat, bis weniger als 20% eine Molekülgröße unterhalb von 200 000 und weniger als 20% eine Molekülgröße oberhalb von 2 000 000 Dalton, bestimmt durch Gelpermeationschromatographie und bezogen auf den Dextran-Standard, aufweisen;

(b) die Aktivierung des erhaltenen kalibrierten Polysaccharids mit Cyanbromid;

(c) die im wesentlichen vollständige Entfernung des unumgesetzten Cyanbromids; und

(d) die Reaktion des aktivierten Produkts mit einem mittels eines Hydrazids einer Dicarbonsäure mit 4 bis 8 Kohlenstoff-Atomen derivatisierten Exotoxoids.

7. Protein- und nucleinsäure-freies Hapten, hergestellt aus kapsulärem Haemophilus Influenzae B Polysaccharid, bestehend aus äquimolaren Mengen Ribose, Ribit und Phosphat, durch Erhitzen, bis weniger als 20% eine Molekülgröße unterhalb von 200 000 und weniger als 20% eine Molekülgröße oberhalb von 2 000 000 Dalton, bestimmt durch Gelpermeationschromatographie und bezogen auf den Dextran-Standard, aufweisen.

8. Hapten nach Anspruch 7, dadurch gekennzeichnet, daß das Polysaccharid als Natrium-Salz vorliegt.

**Revendications**

1. Un conjugué d'exo-anatoxine de polysaccharide d'Haemophilus influenzae b, soluble dans l'eau et thermorésistant, caractérisé en ce que ledit conjugué est capable de provoquer la formation d'anticorps par des cellules T dépendantes en réponse au polysaccharide d'Haemophilus influenzae b, la gamme des tailles moléculaires est supérieure à 140 000 et inférieure à 4 500 000 daltons comme déterminé relativement à un étalon de dextran par chromatographie de filtration sur gel, et le rapport ribose/protéine est entre 0,25 et 1,00, et en ce que ledit conjugué est préparé par mélange d'une anatoxine modifiée par un hydrazide d'acide dicarboxylique de 4—8 atomes de carbone dans une solution exempte d'halogénure de cyanogène avec un polysaccharide capsulaire d'Haemophilus influenzae b, activé par un halogénure de cyanogène, constitué de quantités équimolaires de ribose, de ribitol et de phosphate, qui a été préalablement dimensionné par chauffage jusqu'à ce que plus de 60% du polysaccharide soit ajusté à une taille moléculaire comprise entre 200 000 et 2 000 000 daltons comme déterminé relativement à un étalon de dextran par chromatographie par imprégnation sur gel.

2. Un conjugué selon la revendication 1 dans lequel ledit acide dicarboxylique est l'acide adipique.

3. Un conjugué selon la revendication 1 dans lequel ladite anatoxine est l'anatoxine diphtérique.

4. Un conjugué selon la revendication 1 dans lequel ladite anatoxine est l'anatoxine tétanique.

5. Un conjugué selon la revendication 1 dans lequel ledit halogénure est le bromure.

6. Un procédé pour la production d'un conjugué d'anatoxine selon les revendications 1 à 5, ledit procédé étant caractérisé en ce qu'il comprend:

(a) le chauffage d'un polysaccharide capsulaire d'Haemophilus influenzae b constitué de quantités équimoléculaires de ribose, de ribitol et de phosphate jusqu'à ce que moins de 20% soit d'une taille moléculaire inférieure à 200 000 daltons et moins de 20% soit d'une taille supérieure à 2 000 000 daltons, comme déterminé relativement à un étalon de dextran par chromatographie de filtration sur gel;

(b) l'activation du polysaccharide dimensionné résultant avec du bromure de cyanogène;

(c) l'élimination de la quasi-totalité du bromure de cyanogène n'ayant pas réagi; et

(d) la réaction du produit activé avec une exo-anatoxine modifiée par un hydrazide d'acide dicarboxylique de 4—8 atomes de carbone.

7. Un haptène dépourvu de protéine et d'acide nucléique préparé à partir de polysaccharide capsulaire d'Haemophilus influenzae b, constitué de quantités équimolaires de ribose, de ribitol et de phosphate par chauffage jusqu'à ce que moins de 20% soit d'une taille moléculaire inférieure à 200 000 daltons et moins de 20% soit d'une taille moléculaire supérieure à 2 000 000 daltons comme déterminé relativement à un étalon de dextran par chromatographie de filtration sur gel.

8. Un haptène selon la revendication 7 dans lequel le polysaccharide est présent sous forme du sel de sodium.

**0 098 581**

MICROGRAMS IGG ANTI-PRP PER ML

Groups: GROUP 1, GROUP 2, GROUP 3, GROUP 4, GROUP 5, GROUP 6 (each with P01, P02, P03, P04)